# EUROPEAN PATENT SPECIFICATION

(11) **EP 1 844 712 B1**
(45) Date of publication and mention of the grant of the patent: **16.12.2009**
(21) Application number: 07251393.0
(22) Date of filing: 30.03.2007
(51) Int. Cl.: A61B 10/00, A61B 19/00

(54) **Compression assembly for MRI biopsy device**
Komprimierendes Haltegerät für MRI-Biopsievorrichtung
Dispositif de maintien et de compression pour appareil de biopsie sous IRM

(30) Priority: 31.03.2006 US 395796
(43) Date of publication of application: 17.10.2007
(60) Divisional application: 09075473.0
(73) Proprietor: ETHICON ENDO-SURGERY, INC., Cincinnati, Ohio 45242 (US)
(72) Inventor: Diets, Timothy G., Terrace Park, OH 45174 (US); Clem, William E., Bozeman, MT 59715 (US)
(74) Representative: Tunstall, Christopher Stephen

(56) References cited:
- WO-A-98/50083
- DE-A1- 19 703 418
- DE-A1- 19 709 224
- US-A1- 2005 283 069
- US-B1- 6 244 447

## Description

### Field of the Invention

The present invention relates, in general, to a compression assembly as recited in the preamble of claim 1.

### Background of the Invention

Recently, core biopsy devices have been combined with imaging technology to better target a lesion in breast tissue. One such commercially available product is marketed under the trademark name MAMMOTOME^{™}, by Ethicon Endo-Surgery, Inc. An embodiment of such a device is described in U.S. Patent No. 5,526,822 issued to Burbank, et al., on June 18, 1996. Its handle receives mechanical and electrical power as well as vacuum assist from a remotely positioned control module that is spaced away from the high magnetic field of a Magnetic Resonance Imaging (MRI) machine.

As seen from that reference, the instrument is a type of image-guided, percutaneous coring, breast biopsy instrument. It is vacuum-assisted, and some of the steps for retrieving the tissue samples have been automated. The physician uses this device to "actively" (using the vacuum) capture the tissue prior to severing it from the body. This allows the sampling of tissue of varying hardness. In addition, a side opening aperture is used, avoiding having to thrust into a lesion, which may tend to push the mass away, cause a track metastasis, or cause a hematoma that, with residual contrast agent circulating therein, may mimic enhancement in a suspicious lesion. The side aperture may be rotated about a longitudinal axis of the probe, thereby allowing multiple tissue samples without having to otherwise reposition the probe. These features allow for substantial sampling of large lesions and complete removal of small ones.

Traditionally, prior to biopsy, the clinician immobilizes the breast in a compression system that places light compression on the breast to capture and hold the breast in a static position for the remainder of the procedure. Such compression systems generally consist of two or more compression members that can be adjusted to compress and immobilize the patient's breast. Generally, access to the breast is achieved via fixed slots, grids, or apertures in the compression members. The number of such apertures, and the corresponding number of access points available to the clinician, is limited in order to provide enough surface area to sufficiently restrain the breast during the procedure. Generally, the greater the number of access points preset in a compression member, the less effective the compression member will be in securing the breast during the procedure.

Providing fixed openings through which a biopsy device may be inserted may limit the number of locations through which a clinician may gain access once the compression members are in place. Should a clinician desire access to a location blocked by the compression members, it is generally necessary to reposition the compression members to align the apertures or the like with the desired target area. Rather than reposition the compression members, clinicians may try to work within the limited access areas, thereby potentially decreasing the accuracy or efficacy of the biopsy procedure. Additionally, fixed openings may provide less support than needed in open areas and closed areas may create too high a pressure on narrow areas of the breast.

US 2005/0283069 A1 discloses a compression member which includes positionable and removable bars vertically assembled in a frame DE 197 09 224 A1 discloses a compression member comprising flexible bars horizontally assembled in a frame.

It would therefore be advantageous to provide a compression member for use in biopsy procedures that provides a wide range of access points for a clinician without having to readjust the compression system. It would be further advantageous to provide a compression member that increases the number of available access points to the breast while retaining the ability to effectively hold the breast in place for the duration of the procedure.

### Brief Summary of the Invention

The present invention provides a compression assembly as recited in the claims.

These and other objects and advantages of the present invention shall be made apparent from the accompanying drawings and the description thereof.

### Brief Description of the Figures

The accompanying drawings, which are incorporated in and constitute a part of this specification, illustrate embodiments of compression assemblies as follows:
FIGURE 1 is a perspective view of a Magnetic Resonance Imaging (MRI) biopsy system shown with a patient positioned thereon;
FIGURE 2 is a partial perspective view of the biopsy system of FIG. 1 illustrating one version of a compressiom system and patient support not according to the invention;
FIGURE 3 is a left side longitudinal cross-section view taken along the central axis of the compression system and patient support shown in FIG. 2;
FIGURE 4 is a perspective view of one version of a compression member having multiple compression plates shown prior to use not according to the invention;
FIGURE 5 is a top cross-sectional view, taken along line 5-5, of two compression plates, shown in FIG. 4, and the connection therebetween;
FIGURE 6A is a perspective view of an alternate version of a compression member not according to the invention shown with two compression plates removed;
FIGURE 6B is a perspective view of an alternate version of a compression member not according to the invention shown with slidable compression plates therein;
FIGURE 7 is a top cross-sectional view, taken along line 5-5, of two compression plates, shown in FIG. 6A, and the connection therebetween;
FIGURE 8a is a perspective view of an alternate embodiment of a compression member not according to the invention having horizontal compression bars, shown prior to use;
FIGURE 8b is a perspective view of an alternate embodiment of a compression member not according to the invention having vertical compression bars, shown prior to use;
FIGURE 9 is a top cross-section view, taken along line 9-9, of the interconnection between the compression member and the compression bar of FIG. 8, shown prior to removal;
FIGURE 10 is a top cross-section view, taken along line 9-9, of the interconnection between the compression member and the compression bar of FIG. 8, shown during removal;
FIGURE 11 is a top cross-section view, taken along line 9-9, of an alternate interconnection between the compression member and the compression bar of FIG. 8, shown prior to removal;
FIGURE 12 is a top cross-section view, taken along 9-9, of an alternate interconnection between the compression member and the compression bar of FIG. 8, shown during removal;
FIGURE 13 is a perspective view of an alternate version of a compression assembly not according to the invention having movable members positioned within a frame;
FIGURE 14 is a left side cross-section view, taken along line 14-14, of the compression assembly of FIG. 13;
FIGURE 15 is a top cross-section view, taken along line 15-15, of the compression member of FIG. 13 showing the interconnection between the movable members and the frame;
FIGURE 16 is a perspective view of an alternate version of a compression assembly according to the invention having movable members positioned within a frame;
FIGURE 17 is a front cross-section view, taken along line 17-17, of the compression assembly of FIG. 16, shown depicting the relationship between the movable members and the frame;
FIGURE 18 is a top cross-section view, taken along line 18-18, of the compression assembly of FIG. 16;
FIGURE 19 is a perspective view of an alternate version of a compression assembly not according to the invention having telescoping members positioned within a frame;
FIGURE 20 is a perspective view of an alternate version of a compression assembly not according to the invention having telescoping members positioned within a frame;
FIGURE 21 is a perspective view of an alternate version of a compression assembly not according to the invention having movable members positioned within a frame;
FIGURE 22 is a perspective view of an alternate version of a compression assembly not according to the invention having compression slats configured as a plurality of hexagons positioned within a frame;
FIGURE 23 is a perspective view of an alternate version of a compression assembly not according to the invention having flexible compression bars positioned within a frame;
FIGURE 24 is a perspective view of an alternate version of a compression assembly not according to the invention having removable members positioned within a frame;
FIGURE 25 is a more detailed perspective view of the removable member of FIG. 24 shown with a top insertion member and a bottom insertion member;
FIGURE 26 is a more detailed perspective view of the top insertion member of FIG. 25; and
FIGURE 27 is a more detailed perspective view of the bottom insertion member of FIG. 25.

### Detailed Description of the Invention

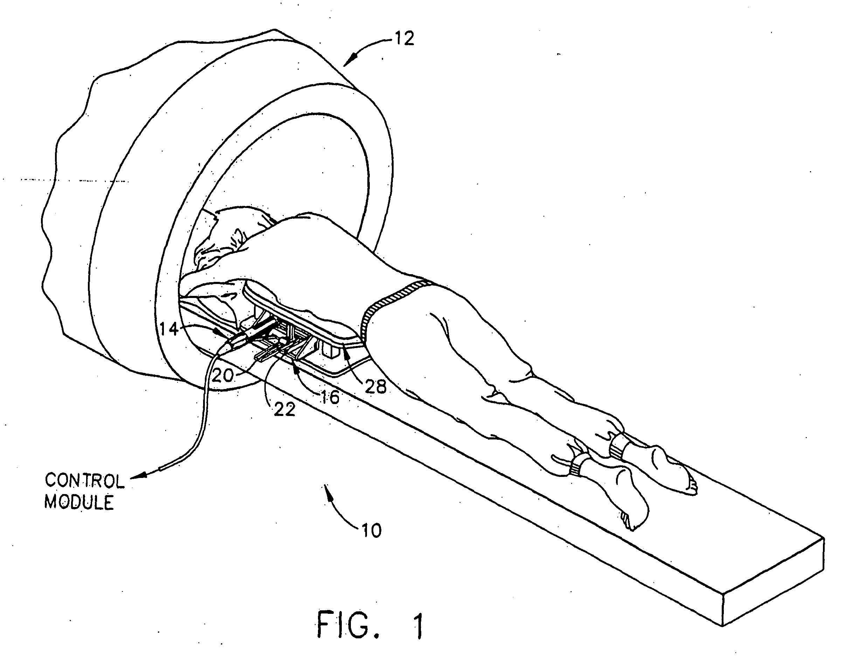

Turning to the Drawings, wherein like numerals denote like components throughout the several views, in FIG. 1, a Magnetic Resonance Imaging (MRI) biopsy system 10, hereinafter biopsy system 10, includes an MRI machine 12, a patient support 28, a localization fixture 16, and a biopsy device 14.

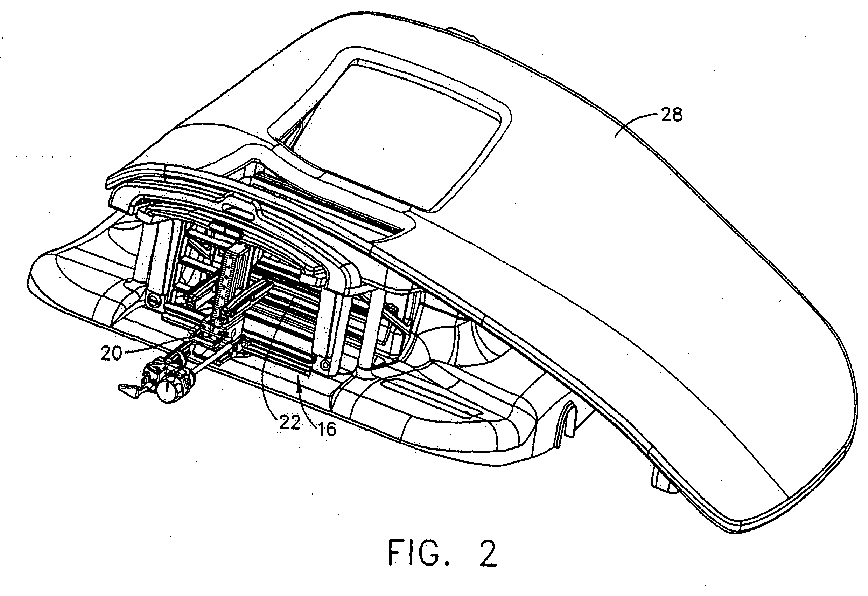

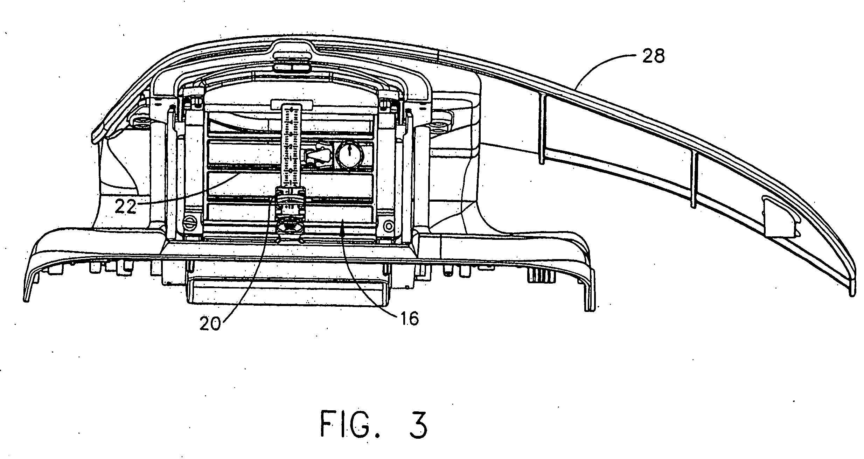

Referring to FIGS. 1-3, the biopsy system 10 includes a control module (not shown) that typically is placed outside of a shielded room containing an MRI machine 12, or at least spaced away, to mitigate detrimental interaction with its strong magnetic field and/or sensitive radio frequency (RF) signal detection antennas. The control module controls and powers the biopsy device 14, which is compatible for use in close proximity to the MRI machine 12. An example of a biopsy device 14 is the aforementioned MAMMOTOME^{™} instrument. The biopsy device 14 is accurately positioned by a localization fixture 16 that is attached to a patient support 28, which supports a patient throughout the duration of the procedure. A guidance assembly 20 may be attached to the localization fixture 16 to increase imaging and therapeutic flexibility and accuracy in conjunction with selective use of the biopsy device 14 at particular parts of the procedure as is known in the art. The guidance assembly 20 may also be mounted or secured separately from the localization fixture 16. In one version, it may be advantageous to mount the guidance assembly 20 independent of the localization fixture 16 where, for example, if a biopsy device 14 having significant mass is utilized it may be advantageous to decouple the targeting and biopsy functions of the biopsy system 10 to allow a clinician to support the biopsy device 14. The localization fixture 16 includes a compression assembly 22 to hold the patient's breast in place and to provide access to the breast during the procedure, versions of which will be discussed in more detail herein.

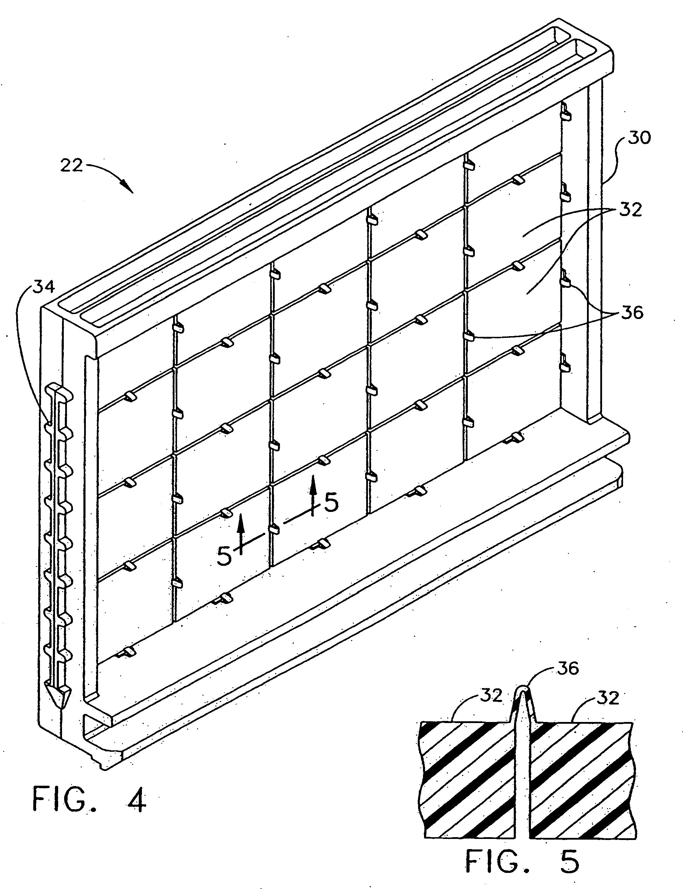

Referring to FIGS. 4-5, one version of a compression assembly 22 is shown having a frame 30 with a plurality of compression members or compression plates 32 located therein. The frame 30 of the compression assembly 22 may be coupled with the localization fixture 16 via one or a plurality of connection members 34 for use during biopsy procedures. The connection members 34 may be configured such that the compression assembly 22 may be adjustable and/or detachable from the localization fixture 16 or other support.

The frame 30 may, for example, be rectangular in shape and have a plurality of removable compression plates 32 positioned therein. In the illustrated version, the compression plates 32 are rectangular in shape and are detachably coupled to adjacent compression plates and/or the frame 30. Referring to FIG. 4, the compression assembly 22 is shown prior to the removal of one or a plurality of compression plates 32. Prior to use, the frame 30 may contain, for example, twenty compression plates 32 of equal size and shape creating a substantially contiguous surface area within the frame 30. The compression plates 32 and the frame 30 may be configured from any material, such as a polycarbonate stabilized for gamma irradiation, suitable for use during an MRI procedure or other medical procedure. It will be appreciated that the frame 30 and/or the compression plates 32 may be configured in any desirable shape or configuration including, but not limited to, circular, oval or three dimensional lofted, curved shapes facilitating access to a patient's breast during a biopsy procedure while still providing sufficient surface area to retain the breast securely therein.

Referring to FIG. 5, the compression plates 32 may be detachably coupled to adjacent compression plates 32 and/or the frame 30 with one or a plurality of disengagable members 36 connected therebetween. The disengagable members 36 may include a polymeric tab, a reattachable clip, a snap-fit between a compression member and another compression member and/or the frame, a frangible coupler, or any other suitable connection mechanism. Although any suitable number is contemplated, each compression plate 32 may have a total of four disengagable members 36 placed one on each side thereof for attachment to an adjacent compression plate 32 and/or frame 30. The disengagable members 36 may be integrally molded with the compression plates 32, attached thereto, for example, with an adhesive or the like, or removable and reattachable. The disengagable members 36 may project outwardly such that a clinician may easily access and remove the disengagable members 36 and/or compression plates 32. Removal may be achieved with the clinician's hands or, for example, with a tool adapted to break, remove, unsnap, or release the disengagable members 36.

Still referring to FIGS. 4-5, in use, the compression system 22 may be placed against the patient's breast with all of the compression plates 32 intact. Once the compression system 22 has been positioned and secured, the clinician may identify the region on the plate through which access to the breast would be most desirable. After identifying this area, the clinician may remove one or a plurality of compression plates 32 in this area by breaking or otherwise removing the disengagable members 36. Removing, for example, the four disengagable members 36 surrounding a compression plate 32 may release the compression plate 32 for removal by the clinician. Once the compression plate 32 has been removed, the clinician may access the breast with the biopsy device 14 via the newly created access point. In one version, the compression plate 32 may be reattached to the compression assembly 22 after the biopsy sample is taken.

Providing a compression assembly 22 in accordance with the illustrated version may provide a clinician with nearly unlimited access to a patient's breast within the frame 30. Once a targeted area has been identified, a clinician may avoid having to readjust major components in order to have direct access to a desirable tissue region. A clinician may only need to remove those compression plates 32 from the compression assembly 22 that correspond to the targeted tissue location before inserting the biopsy device 14 therethrough. Additionally, the compression assembly 22 may more securely retain the patient's breast by allowing a clinician to create gaps or apertures in only those areas through which access is needed. Similarly, high levels of pressure on narrow regions of breast tissue may be avoided by provided a large surface area in areas through which access to the breast is not desired. Removing only necessary portions of the available surface area may increase the breast retention capabilities of the compression assembly 22 while simultaneously providing greater access to the patient's breast. Increasing a clinician's access to breast tissue while simultaneously securing the breast with an increased surface area may improve the accuracy and ease of biopsy procedures. It will be appreciated that versions of the compression assemblies and/or compression members disclosed herein may be operably configured for single use or for multiple use.

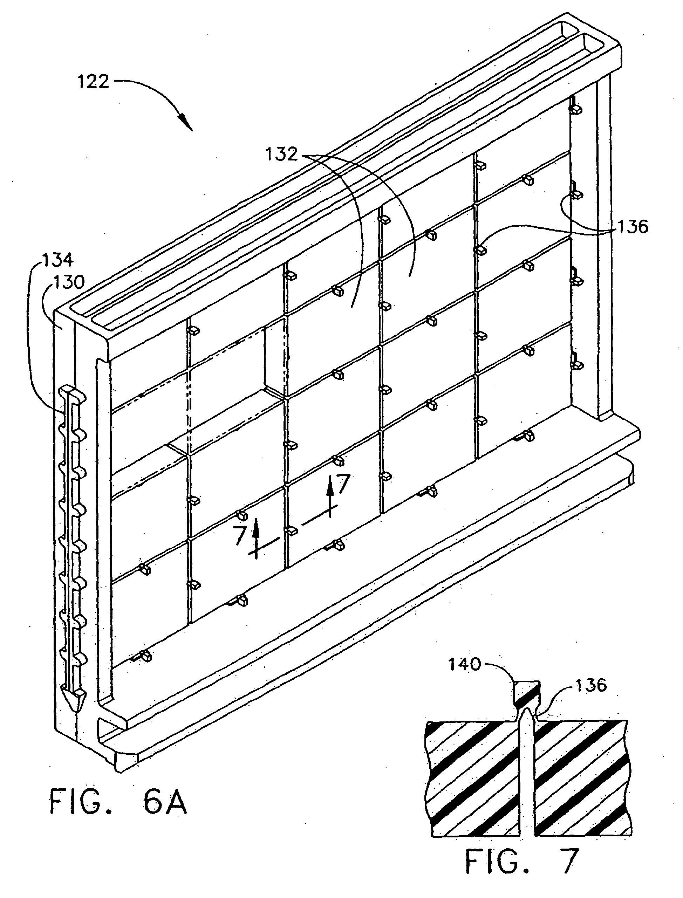

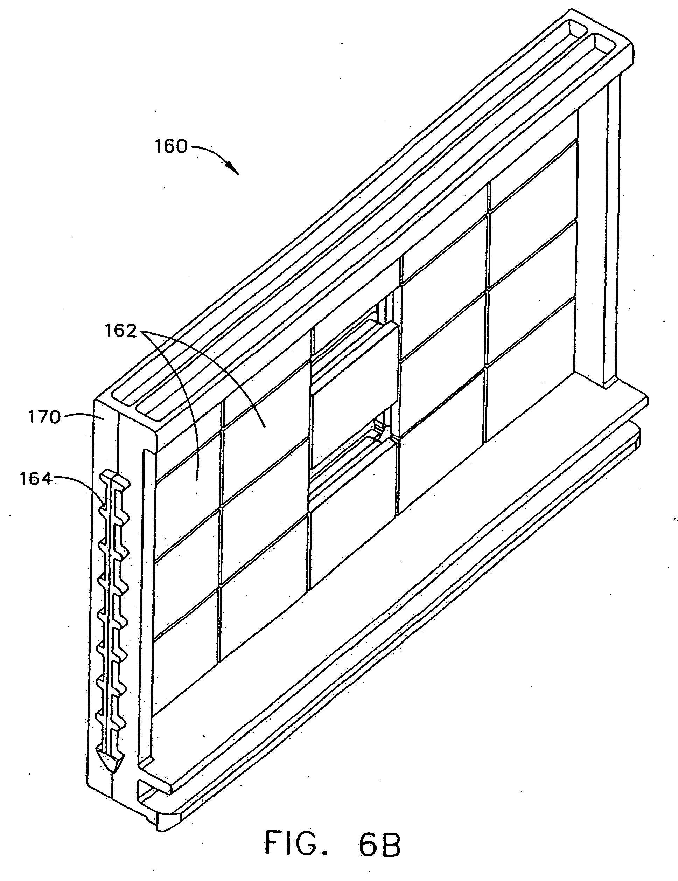

Referring to FIGS. 6-7, an alternate version of a compression assembly 122 is shown with two compression members or compression plates 132 removed from the frame 130. The frame 130 of the compression assembly 122 may be coupled with the localization fixture 16 via one or a plurality of connection members 134 for use during biopsy procedures. The connection members 134 may be configured such that the compression assembly 122 may be adjustable and/or detachable from the localization fixture 16 or other support.

The frame 130 may, for example, be rectangular in shape and have a plurality of removable compression plates 132 positioned therein. In the illustrated version, the compression plates 132 are rectangular in shape and are detachably coupled to adjacent compression plates and/or the frame 130. Referring to FIG. 6a, the compression assembly 122 is shown after the removal of two compression plates 132. Prior to use, the frame 130 may contain, for example, twenty compression plates 132 of equal size and shape creating a substantially contiguous surface area within the frame 130. During use, as illustrated, one or a plurality of compression plates 132 may be removed to facilitate access to the patient's breast. The compression plates 132 and the frame 130 may be configured from any material, such as a polycarbonate stabilized for gamma irradiation, suitable for use during an MRI procedure or other medical procedure. It will be appreciated that the frame 130 and/or the compression plates 132 may be configured in any desirable shape or configuration facilitating access to a patient's breast during a biopsy procedure.

Referring to FIG. 7, the compression plates 132 may be detachably coupled to adjacent compression plates 132 and/or the frame 130 with a disengagable member 136 connected or otherwise creating a connection therebetween. The disengagable members 136 may include, for example, a tab 140 that may be easily grasped and removed manually by a clinician. Although any suitable number is contemplated, each compression plate 132 may have a total of four disengagable members 136 placed one on each side thereof for attachment to an adjacent compression plate 132 and/or frame 130. The disengagable members 136 may be integrally molded with the compression plates 132 or may be attached thereto, for example, with an adhesive or the like. The disengagable members 136 may project outwardly such that a clinician may easily access and remove the disengagable members 136 and/or compression plates 132. Removal may be achieved with the clinician's hands or, for example, with a tool adapted to break, unsnap, disengage, and/or remove the disengagable members 136.

Referring to FIG. 6b, an alternate version of a compression assembly 160 is shown having a frame 170 with a plurality of compression members or compression plates 162 located therein. The frame 170 of the compression assembly 160 may be coupled with the localization fixture 16 via one or a plurality of connection members 164 for use during biopsy procedures. The connection members 164 may be configured such that the compression assembly 160 may be adjustable and/or detachable from the localization fixture 16 or other support.

The frame 170 may, for example, be rectangular in shape and have a plurality of slidable or movable compression plates 162 positioned therein. In the illustrated version, the compression plates 162 are rectangular in shape and are coupled to adjacent compression plates and/or the frame 170 with a slidable tongue and groove configuration. Referring to FIG. 6b, the compression assembly 160 is shown after a compression plate 162 has been moved within the frame 170 by sliding the compression plate 162 within a vertical plane along a tongue and groove created by adjacent compression plates 162. Each compression plate 162 and/or the frame 170 may include tongues and/or grooves such that compression plates 162 may be moved freely in a vertical and/or horizontal direction within the frame 170.

Prior to use, the frame 170 may contain, for example, nineteen compression plates 162 of equal size and shape creating a substantially contiguous surface area within the frame 170 minus an opening the size of a single compression plate. Using the slidable tongue and groove connection between adjacent compression plates 162 and/or the frame 170, the clinician may adjust, slide, or otherwise move the compression plates about one another until the gap created by the absent compression plate is positioned as an access point inline with a targeted tissue location. Providing a movable access point may allow a clinician to access a wide variety of tissue locations while preserving sufficient surface area to retain the breast. It will be appreciated that the compression assembly 160 may include any suitable number of compression members or compression plates 162 and may have a movable gap, aperture, or access point of any desirable size and configuration. It is further contemplated, for example, that two or more compression plates may be removed such that multiple access points may be created simultaneously.

The compression plates 162 and the frame 170 may be configured from any material, such as a polycarbonate stabilized for gamma irradiation, suitable for use during an MRI procedure or other medical procedure. It will be appreciated that the frame 170 and/or the compression plates 162 may be configured in any desirable shape or configuration facilitating access to a patient's breast during a biopsy procedure. The movement of the compression plates 162 may be facilitated by, for example, a tongue and groove relationship between adjacent compression plates 162 and/or the frame 170, or by any other suitable adjusting means.

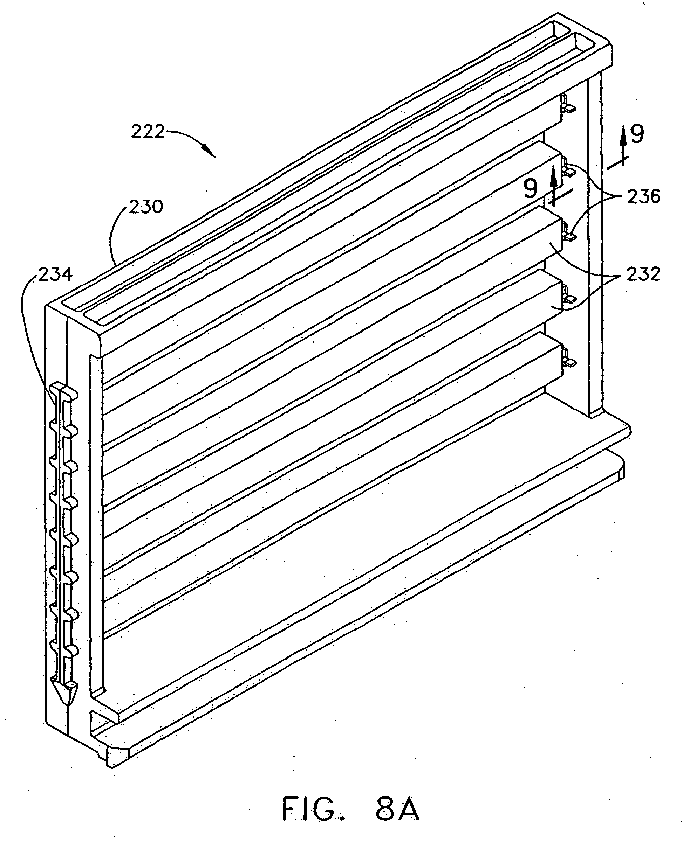

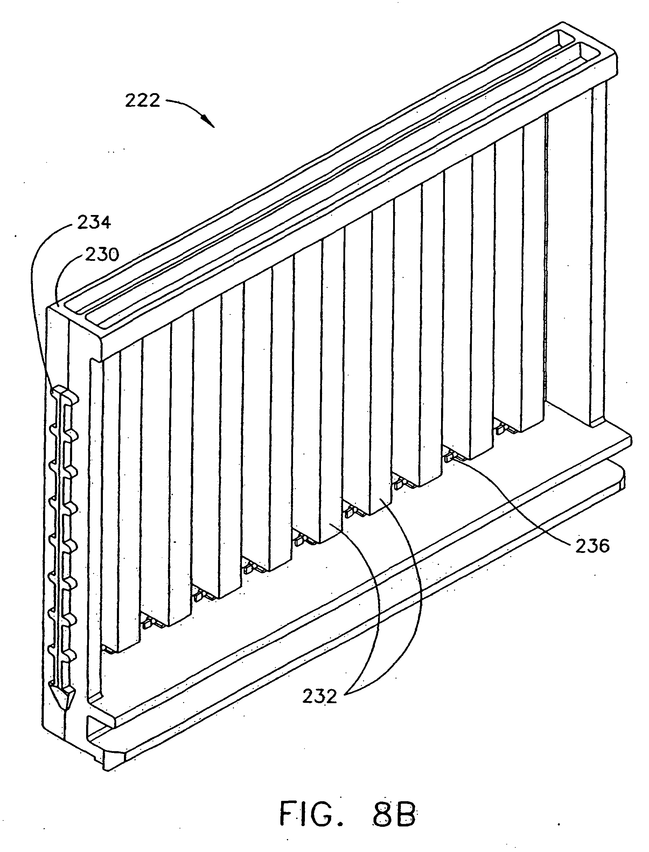

Referring to FIGS. 8-12, an alternate version of a compression assembly 222 is shown having a frame 230 with a plurality of compression members or compression bars 232 positioned therein. The frame 230 of the compression assembly 222 may be coupled with the localization fixture 16 via one or a plurality of connection members 234 for use during biopsy procedures. The connection members 234 may be configured such that the compression assembly 222 may be adjustable and/or detachable from the localization fixture 16 or other support.

The frame 230 may, for example, be rectangular in shape and have a plurality of removable compression bars 232 positioned therein. In the illustrated version, the compression bars 232 are rectangular in shape and are detachably coupled to the frame 230 at both ends. Referring to FIGS. 8a-8b, the compression assembly 222 is shown prior to the removal of a compression bar 232. In one version, the frame 230 may contain, for example, five compression bars 232 of equal size and shape spaced apart such that access to the breast is available without removing one or a plurality of the compression bars 232. Should a clinician desire greater access, one or a plurality of the compression bars 232 may be removed to grant such access. Providing established access points, in combination with the ability to create additional access points, may allow a clinician to perform a procedure quickly without modifying the compression assembly 222 unless necessary. The compression bars 232 and the frame 230 may be configured from any material, such as a polycarbonate stabilized for gamma irradiation, suitable for use during an MRI procedure or other medical procedure. It will be appreciated that the frame 230 and/or the compression bars 232 may be configured in any desirable shape or configuration such as, for example, horizontally, vertically, diagonally, ergonomically, curved, and/or in any suitable shape that models or fits the patient's anatomy. It is further contemplated that any suitable number of compression bars 232 may be provided. Referring to FIG. 8a, one version of a compression assembly 222 is shown having horizontal compression bars 232. Referring to FIG. 8b, one version of a compression assembly 222 is shown having vertical compression bars 232.

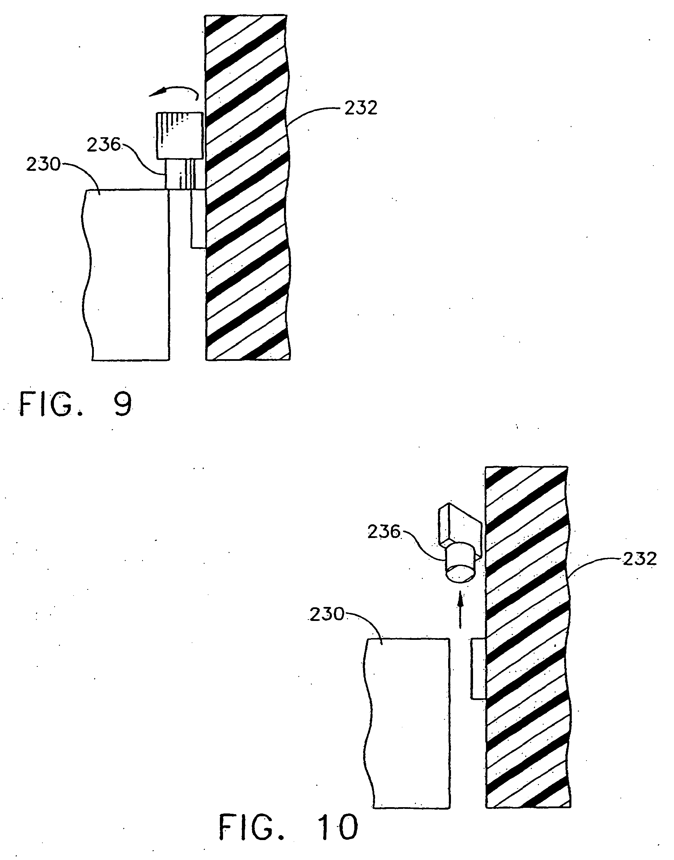

Referring to FIGS. 9-10, the compression bars 232 may be detachably coupled to the frame 230 with a disengagable member 236 connected or otherwise providing a connection therebetween. The disengagable members 236 may be polymeric tabs or any other suitable coupling or attachment mechanism. Although any suitable number is contemplated, each compression bar 232 may have a single disengagable member 236 at each end thereof. The disengagable members 236 may be integrally molded with the compression bars 232 or may be attached thereto, for example, with an adhesive, a snap fit, or the like. The disengagable members 236 may project outwardly such that a clinician may easily access and remove the disengagable members 236 and/or the compression bars 232. Removal may be achieved with the clinician's hands or, for example, with a tool adapted to break, disengage, remove, unsnap, or otherwise uncouple the disengagable members 236. It is further contemplated that the compression bars 232 may be reattachable to the frame 230.

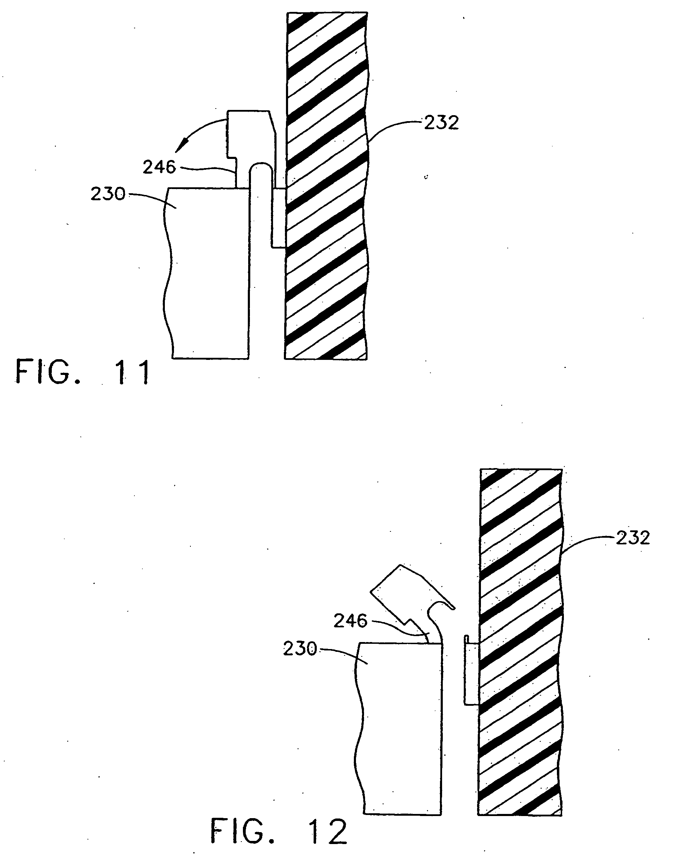

Referring to FIGS. 11-12, an alternate version of a disengagable member 246 is shown prior to the removal of a compression bar 232 (FIG. 11) and during removal of a compression bar 232 (FIG. 12). In the illustrated version, the disengagable member 246 may be permanently coupled to the frame 230 and is detachable only from the compression bar 232 by breaking the disengagable member 246. A clinician, for example, by breaking the disengagable member 246, may remove the compression bar 232 without having to dispose ofa loose disengagable member 246. In one version, the connection of the disengagable member 246 to the frame 230 may be thicker, or otherwise more secure, than the connection of the disengagable member 246 to the compression bar 232. When the disengagable member 246 is twisted, bent, toggled, or the like by the clinician, the connection between the disengagable member 246 and the compression bar 232 may be such that it is broken, thereby releasing the compression bar 232 without creating a loose disengagable member 246.

It will be appreciated that the disengagable member 246 is disclosed by way of example where, for example, the disengagable member 246 may be more securely attached to the compression bar than to the frame 230 such that the disengagable member 246 remains attached to the compression bar 232 when the seal, tab, clip, connection, or other suitable connection mechanism is broken or removed. It will be further appreciated that versions of the frangible or disengagable members disclosed herein may be applied to any suitable version of the compression assembly. It is further contemplated that disengagable members disclosed herein may be reattached to the compression assemblies such that, if desired, the clinician may reattach a compression bar, plate, slat, movable member, or the like to the compression assembly if desired.

Referring to FIGS. 8-12, in use, the compression assembly 222 may be placed against the patient's breast with all of the compression bars 232 intact. Once the compression system 222 has been positioned and secured, the clinician may identify the region within the frame 230 through which access to the breast would be most desirable. If this area is already open via the established access ports, the clinician may proceed with the biopsy procedure without removing any of the components of the compression assembly 222. If the desired access area is blocked by a compression bar 232, the clinician may remove one or a plurality of the compression bars 232 in the target area by breaking or removing the disengagable members 236. Removing, for example, the two disengagable members 236 at the ends of a compression bar 232 may release the compression bar 232 for removal by the clinician. Once the compression bar 232 has been removed, the clinician may access the breast with the biopsy device 14 via the newly created access point.

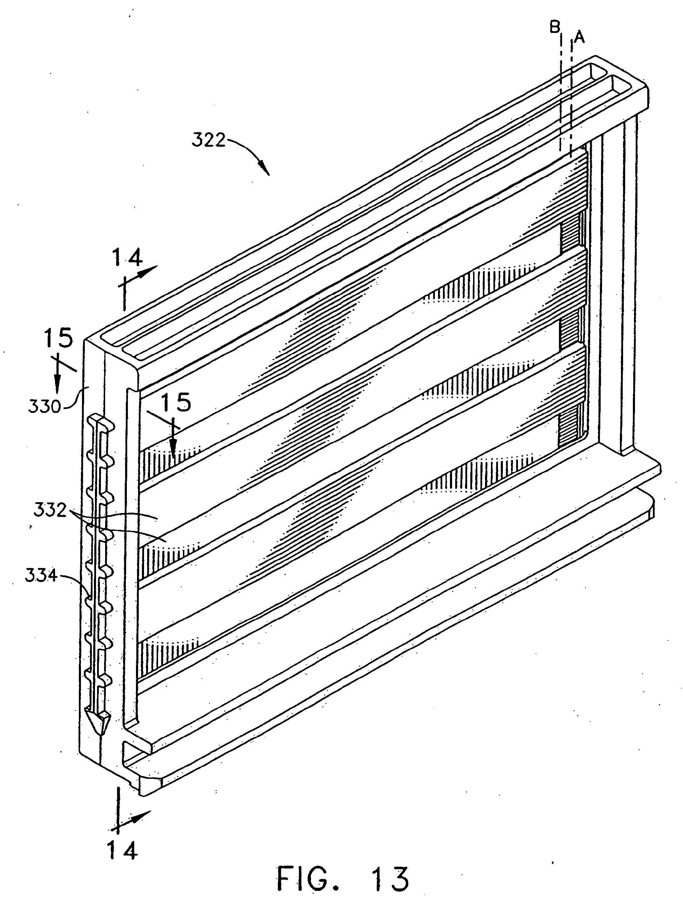

Referring to FIGS. 13-15, an alternate version of a compression assembly 322 is shown having a frame 330 with a plurality of compression members or movable members 332 positioned therein. The frame 330 of the compression assembly 322 may be coupled with the localization fixture 16 via one or a plurality of connection members 334 for use during biopsy procedures. The connection members 334 may be configured such that the compression assembly 322 may be adjustable and/or detachable from the localization fixture 16 or other support.

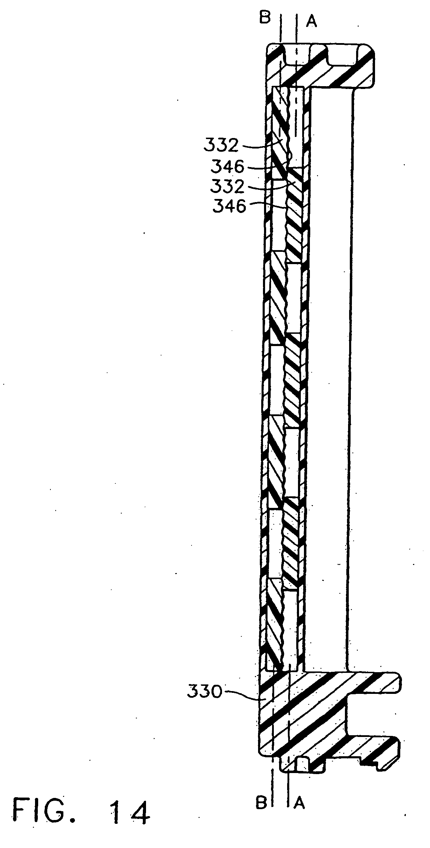

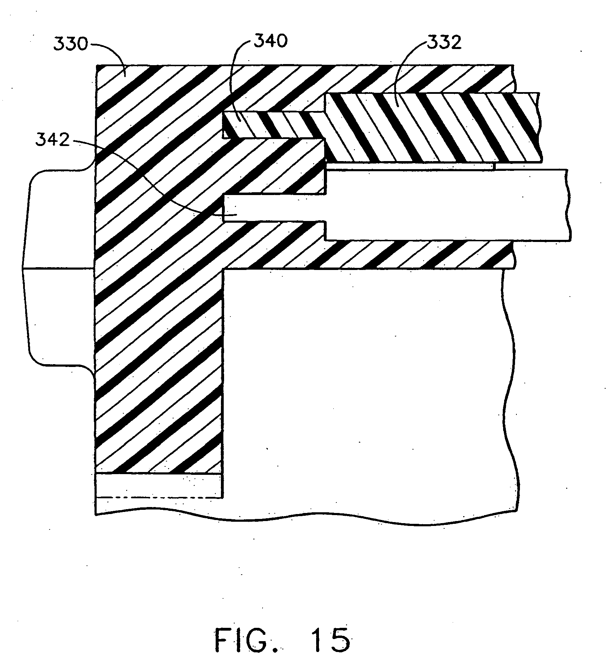

The frame 330 may, for example, be rectangular in shape and have a plurality of compression members or movable members 332 positioned therein. In the illustrated version, the movable members 332 are rectangular slats slidably or adjustably coupled to the frame 330 at both ends in a tongue and groove. Referring to FIGS. 13-15, the compression assembly 322 is shown prior to the adjustment of one or a plurality of movable members 332. In one version, the frame 330 may contain, for example, seven movable members 332 of equal size and shape staggered in an alternating configuration (FIG. 14) where, for example, four movable members 332 are adjustable or slidable about a plane A-A and three movable members are adjustable or slidable about a plane B-B. Referring to FIG. 15, the four movable members 332 adjustable about plane A-A may ride, in a tongue and groove fashion, within a first track 340 parallel to plane A-A. The three movable members 332 adjustable about plane B-B may ride, in a tongue and groove fashion, within a second track 342 parallel to plane B-B. The movable members 332 may be freely adjustable about the planes A-A and B-B or may be held in place once positioned, for example, with a friction fit between adjacent surfaces 346 of the movable members 332 or a ratchet system.

When initially provided, the compression assembly 322 may lack visible apertures or holes through which a clinician may access the patient's breast. After identifying a target area to which access is desired, one or a plurality of the movable members 332 may be adjusted, moved, and/or removed to provide a suitable opening through which a biopsy device 14 may be inserted. By adjusting the movable members 332 about planes A-A and B-B, the clinician may access a variety of regions within the bounds of the frame 330. The movable members 332 may be adjusted by any suitable mechanism including, for example, by manual adjustment or with a tool adapted for that purpose. The movable members 332 and the frame 330 may be configured from any material, such as a polycarbonate stabilized for gamma irradiation, suitable for use during an MRI procedure or other medical procedure. It will be appreciated that the frame 330 and/or the movable members 332 may be configured in any desirable shape, thickness, or configuration such as, for example, with diagonally, horizontally, and/or vertically positioned movable members 332. It is further contemplated that any suitable number of movable members 332 may be provided that may be staggered about any suitable number of planes.

Providing a compression assembly 322 with movable members 332 may allow a clinician to access the breast through a wide range of areas within the frame 330. A clinician may access a desired area while retaining sufficient support for the breast by moving only those movable members 332 necessary to create the access point.

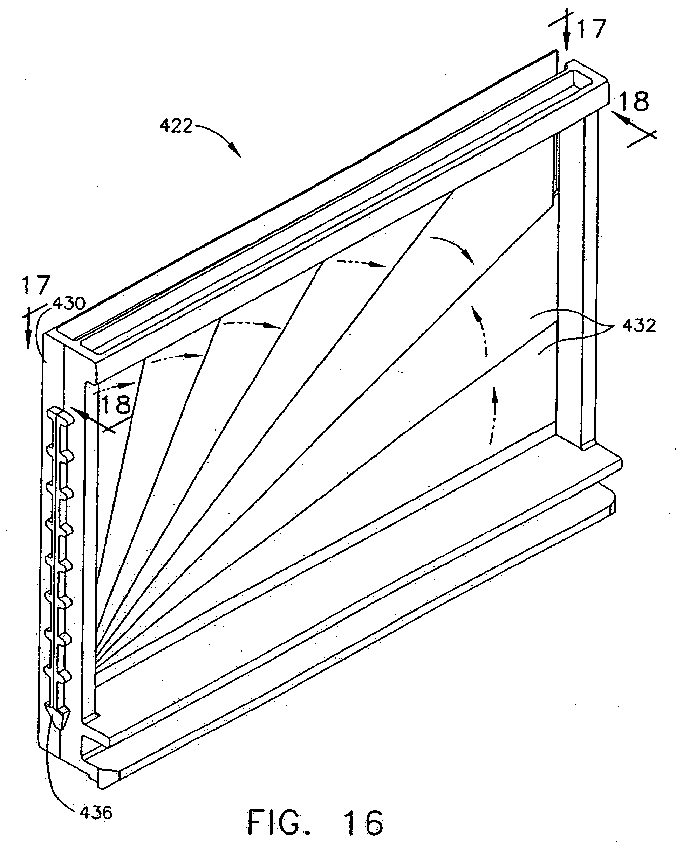

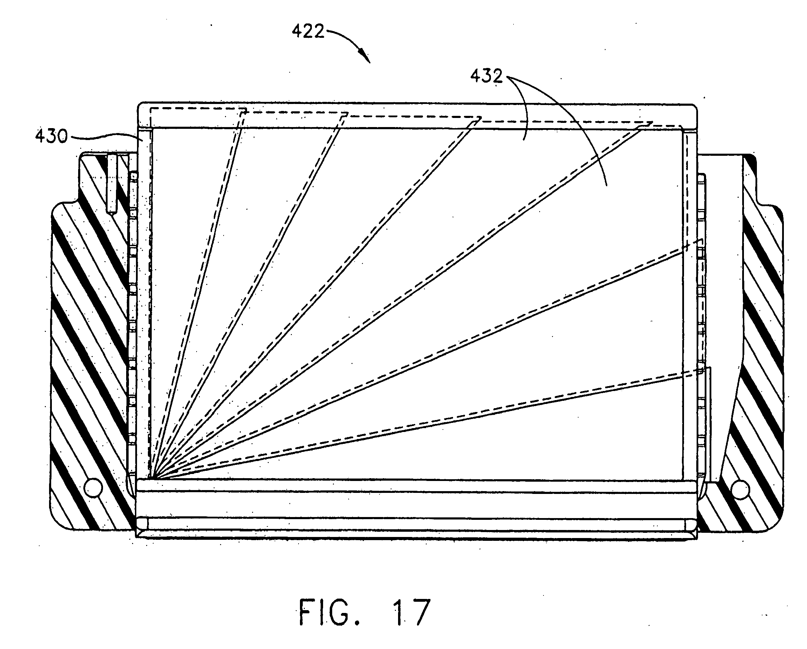

Referring to FIGS. 16-18, an alternate version of a compression assembly 422 is shown having a frame 430 with a plurality of compression members or movable members 432 positioned therein. The frame 430 of the compression assembly 422 may be coupled with the localization fixture 16 via one or a plurality of connection members 434 for use during biopsy procedures. The connection members 434 may be configured such that the compression assembly 422 may be adjustable and/or detachable from the localization fixture 16 or other support.

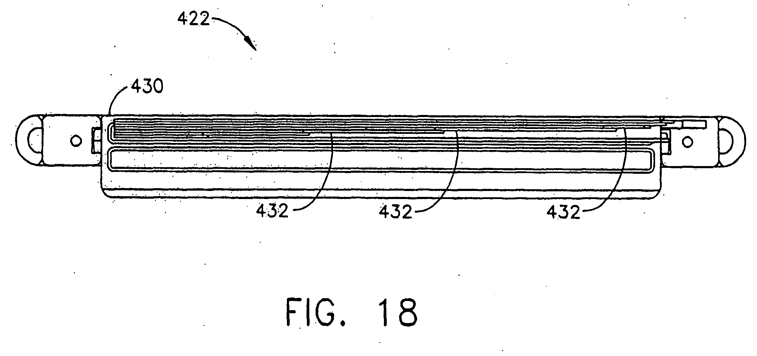

The frame 430 may, for example, be rectangular in shape and have a plurality of movable members 432 positioned therein. In the illustrated version, the movable members 432 are fan-shaped slats slidably or adjustably coupled, at one end, to the frame 430 with a hinge or the like. Referring to FIGS. 16-18, the compression assembly 422 is shown prior to the adjustment of one or a plurality of movable members 432. In one version, the frame 430 may contain, for example, seven movable members 332 configured in the shape of a fan and stacked upon one another (FIG. 18) where, for example, each movable member 432 is slidable or adjustable about a separate plane running parallel to the front face of the frame 430. The movable members 432 may be freely adjustable about the plurality of planes and may be held in place once positioned, for example, with a friction fit between adjacent surfaces of the movable members 432 or by any other suitable means.

When initially provided, the compression assembly 422 may not have any visible apertures or holes through which a clinician may access the patient's breast. After identifying a target area to which access is desired, one or a plurality of the movable members 432, may be adjusted or moved such as, for example, by rotating the movable member 432 about an axis or hinge located in one corner of the frame 430. By rotating the movable members 432, the clinician may access a wide variety of locations within the bounds of the frame 430. The movable members 432 may be adjusted by any suitable means including, for example, by manual adjustment or with a tool adapted for such a purpose. The movable members 432 and the frame 430 may be configured from any material, such as a polycarbonate stabilized for gamma irradiation, suitable for use during an MRI procedure or other medical procedure. It will be appreciated that the frame 430 and/or the movable members 432 may be configured in any desirable shape, thickness, or configuration. It is further contemplated that any suitable number of movable members 432 may be provided. Generally, it will be appreciated that the compression assemblies disclosed herein may be used laterally, medially, or otherwise. It is further contemplated that versions herein may be combined such as, for example, by providing movable members that may also be released with a disengagable member or the like.

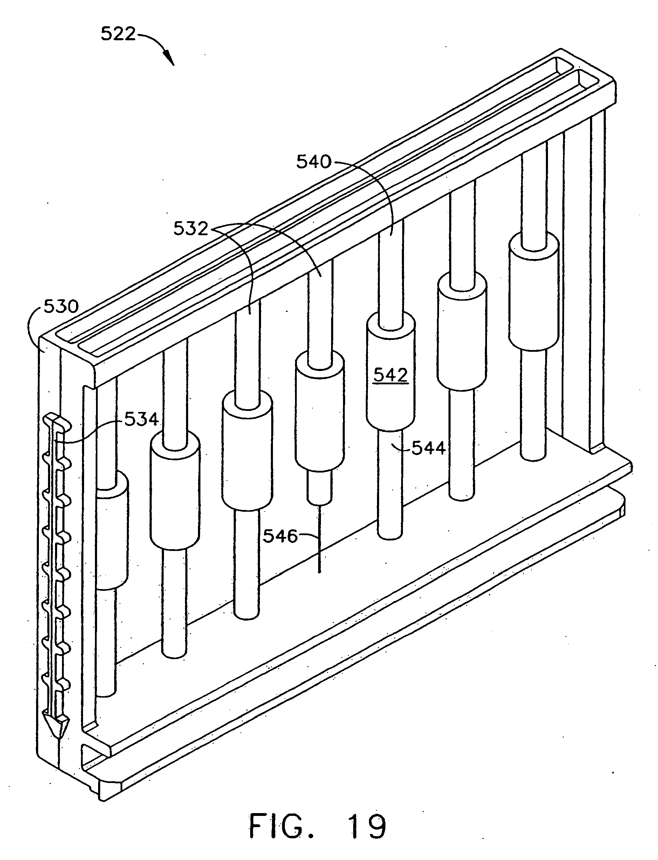

Referring to FIGS. 19-20, versions of a compression assembly 522 are shown having a frame 530 with a plurality of telescoping compression members or compression bars 532 located therein. The frame 530 of the compression assembly 522 may be coupled with the localization fixture 16 via one or a plurality of connection members 534 for use during biopsy procedures. The connection members 534 may be configured such that the compression assembly 522 may be adjustable and/or detachable from the localization fixture 16 or other support.

The frame 530 may, for example, be rectangular in shape and have a plurality of telescoping compression members or compression bars 532 positioned therein. In the illustrated version, the telescoping compression bars 532 are cylindrical in shape, are vertically positioned, and include a first telescoping member 540, a second telescoping member 542, and a third telescoping member 544. The frame 530 may contain, for example, seven telescoping compression bars 532 of equal size and shape spaced apart such that the breast is adequately supported during an MRI procedure. The telescoping compression bars 532 and the frame 530 may be configured from any material, such as a polycarbonate stabilized for gamma irradiation, suitable for use during an MRI procedure or other medical procedure. It will be appreciated that the frame 530 and/or the telescoping compression bars 532 may be configured in any desirable shape or configuration facilitating access to a patient's breast during a biopsy procedure while still providing sufficient surface area to retain the breast securely therein.

Still referring to FIGS. 19-20, the first, second, and third telescoping members 540, 542, 544 may telescope upon, within, and/or about one another such that a desired access region is exposed. The first, second, and third telescoping members 540 may be vertically movable, for example, about a guidewire 546 connected at each end thereof to the frame 530. The telescoping members 540, 542, 544 may have a central bore through which the guidewire 546 passes and about which the telescoping members 540, 542, 544 may be adjusted. The telescoping members 540, 542, 544 may be operably configured such that a friction fit, or any other suitable connection, maintains the telescoping members 540, 542, 544 in a static position until moved. Once moved or adjusted, the friction fit may maintain the position of the telescoping members 540, 542, 544 in the desired position. In one version, the telescoping members 540, 542, 544 have compatible diameters such that, for example, all three telescoping members 540, 542, 544 may be stacked upon one another, at any point along the guidewire 546, to provide a wide range of access to the patient's breast. It is further contemplated that the telescoping members 540, 542, 544 be tapered or the like to reduce the likelihood of pinching the patient's skin.

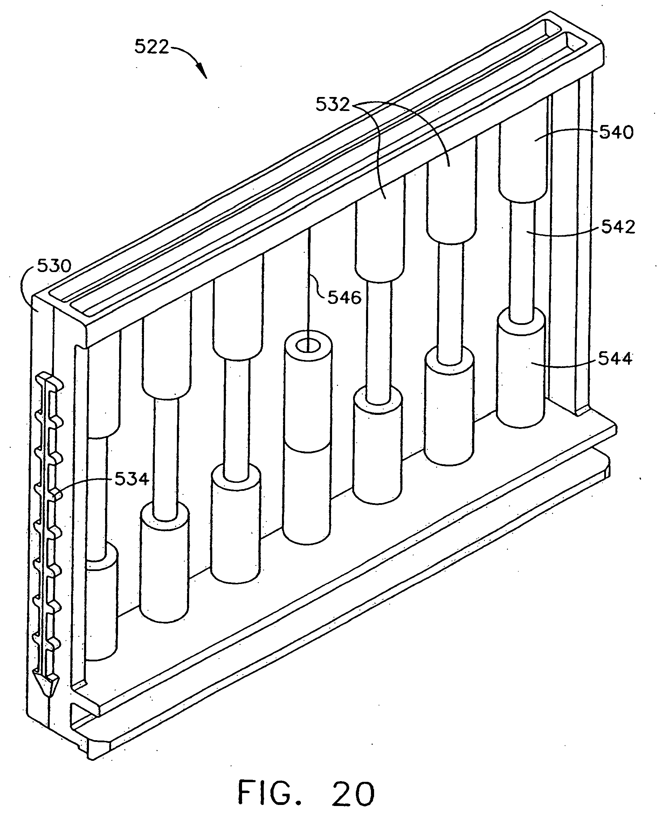

Still referring to FIGS. 19-20, the telescoping members 540, 542, 544 may be provided in any suitable configuration that allows for access to a patient's breast. For example, as illustrated in FIG. 19, the second telescoping member 542 may have a relatively wide diameter configured to accept the first and third telescoping members 540, 544, therein. Referring to FIG. 20, an alternate version is shown wherein the first and second telescoping members 540, 544 are provided with a relatively wide diameter configured to accept the second telescoping member 542 therein. It will be appreciated that the telescoping compression bars 532 may have any suitable configuration and/or any suitable telescoping or adjustable capabilities to provide a clinician with desirable access to a patient's breast. For example, any suitable number of telescoping members is contemplated in both a vertical or horizontal configuration. Furthermore, if a guidewire 546 is used, the tension of the guidewire 546 may be varied to allow the telescoping members 542 or knuckles to conform to the shape of the patient's anatomy. It is further contemplated that the telescoping compression bars 532 may be self-guiding in the absence of a guidewire 546.

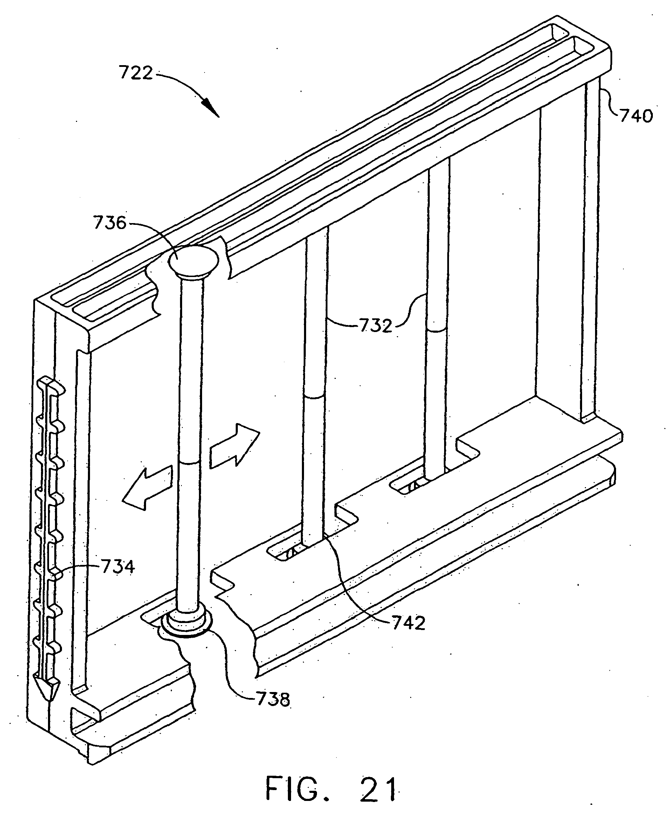

Referring to FIG. 21, one version of a compression assembly 722 is shown having a frame 730 with a plurality of compression members or compression bars 732 located therein. The frame 730 of the compression assembly 722 may be coupled with the localization fixture 16 via one or a plurality of connection members 734 for use during biopsy procedures. The connection members 734 may be configured such that the compression assembly 722 may be adjustable and/or detachable from the localization fixture 16 or other support.

The frame 730 may, for example, be rectangular in shape and have a plurality of adjustable compression bars 732 positioned therein. In the illustrated version, the adjustable compression bars 732 are cylindrical in shape, are vertically positioned, and are operably configured such that they may be moved horizontally and/or rotated within the frame 730. Horizontal motion may open advantageous access points to the patient's breast and rotation may minimize the likelihood of pinching the patient's skin. The frame 730 may contain, for example, three adjustable compression bars 732 of equal size and shape spaced apart such that the breast is adequately supported during an MRI procedure. The adjustable compression bars 732 and the frame 730 may be configured from any material, such as a polycarbonate stabilized for gamma irradiation, suitable for use during an MRI procedure or other medical procedure. It will be appreciated that the frame 730 and/or the adjustable compression bars 732 may be configured in any desirable shape or configuration facilitating access to a patient's breast during a biopsy procedure while still providing sufficient surface area to retain the breast securely therein.

Still referring to FIG. 21, the adjustable compression bars 732 may be rotatably and/or movably coupled to the frame 730 with a first adjustable retention member 736 at one end and a second adjustable retention member 738 at the opposite end. The adjustable retention members 736, 738 may be operably configured to ride within a first track 740 and a second track 742, respectively. In one version, the adjustable retention members are operably configured to be adjusted should a physician desire access to a region of the breast temporarily blocked. The compression bars 732 may be rotated to minimize movement of the breast and to maximize the biopsy accuracy.

In a further version, the adjustable compression bars 732 may be rotated where, for example, the compression bar 732 may have different geometries based upon its orientation with respect to the central axis. For example, the compression bar 732 may have a slat-like shape with one face having a large surface area and a second face having a low surface area. When a clinician does not need access to the breast near the compression bar 732, the compression bar 732 may be rotated such that the face having a high surface area is pushed against the breast in order to provide a secure hold. Should a clinician desire to take a biopsy sample in the area of the compression bar 732, the compression bar 732 may be rotated or the like to turn the face having a low surface area against the breast to give the clinician greater access. It will be appreciated that the compression bar 732 may have any suitable configuration and/or any suitable rotational or movement capabilities to provide a clinician with desirable access to a patient's breast.

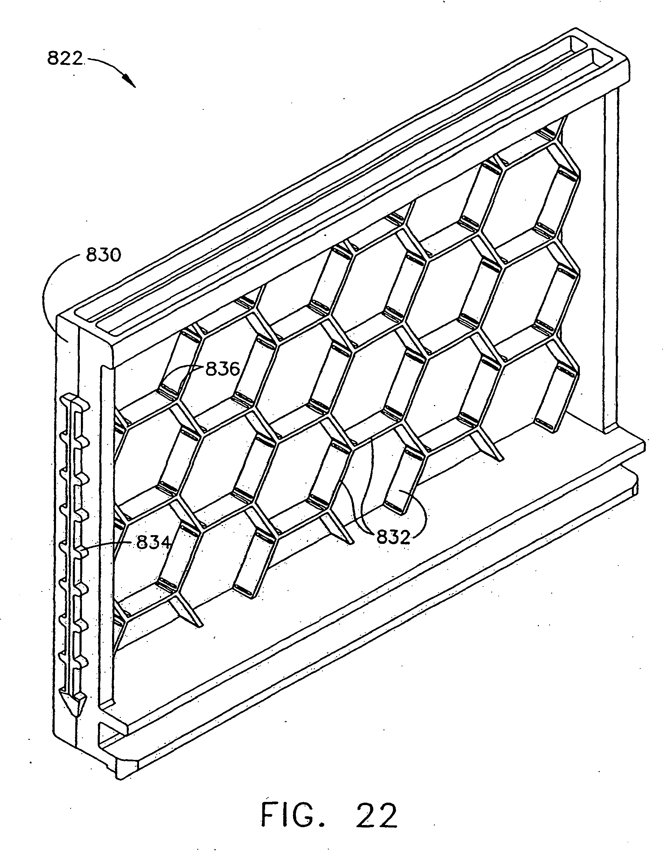

Referring to FIG. 22, one version of a compression assembly 822 is shown having a frame 830 with a plurality of compression members or compression slats 832 located therein. The frame 830 of the compression assembly 822 may be coupled with the localization fixture 16 via one or a plurality of connection members 834 for use during biopsy procedures. The connection members 834 may be configured such that the compression assembly 822 may be adjustable and/or detachable from the localization fixture 16 or other support.

The frame 830 may, for example, be rectangular in shape and have a plurality of removable compression slats 832 positioned therein. In the illustrated version, the compression slats 832 are rectangular in shape and are detachably coupled to adjacent compression plates and/or the frame 830. Still referring to FIG. 22, the compression assembly 822 is shown prior to the removal of one or a plurality of compression slats 832. Prior to use, the frame 830 may contain, for example, a plurality of compression slats 832 of equal size and shape organized into a plurality of hexagons, however, it will be appreciated that any organization is contemplated. The compression slats 832 and the frame 830 may be configured from any material, such as a polycarbonate stabilized for gamma irradiation, suitable for use during an MRI procedure or other medical procedure. It will be appreciated that the frame 830 and/or the compression slats 832 may be configured in any desirable shape or configuration, such as a configuration designed to conform to a patient's anatomy, facilitating access to a patient's breast during a biopsy procedure while still providing sufficient surface area to retain the breast securely therein.

Still referring to FIG. 22, the compression slats 832 may be detachably coupled to adjacent compression slats 832 and/or the frame 830 with a disengagable member 836 connected therebetween. The disengagable members 836 may be a polymeric tab, a reattachable clip, a snap-fit between a compression member and another compression member and/or the frame, a frangible coupler, or any other suitable connection mechanism. Although any suitable number is contemplated, each compression slat 832 may have a disengagable member 36 at each end thereof for attachment to an adjacent compression slat 832 and/or frame 830. The disengagable members 836 may be integrally molded with the compression slats 832, attached thereto, for example, with an adhesive or the like, or removable and reattachable. In one version, the disengagable members 836 may project outwardly such that a clinician may easily access and remove the disengagable members 836 and/or compression slats 832. Removal may be achieved with the clinician's hands or, for example, with a tool adapted to break, remove, unsnap, or release the disengagable members 836.

Still referring to FIG. 22, in use, the compression system 822 may be placed against the patient's breast with all of the compression slats 832 intact. Once the compression system 822 has been positioned and secured, the clinician may identify the region on the plate through which access to the breast would be most desirable. After identifying this area, the clinician may remove one or a plurality of compression slats 832 in this area by breaking or otherwise removing the disengagable members 836. Removing, for example, the two disengagable members 836 at both ends of a compression slat 832 may release the compression slat 832 for removal by the clinician. Once the compression slat 832 has been removed, the clinician may access the breast with the biopsy device 14 via the newly created access point. In one version, the compression slat 832 may be reattached to the compression assembly 822 after the biopsy sample is taken.

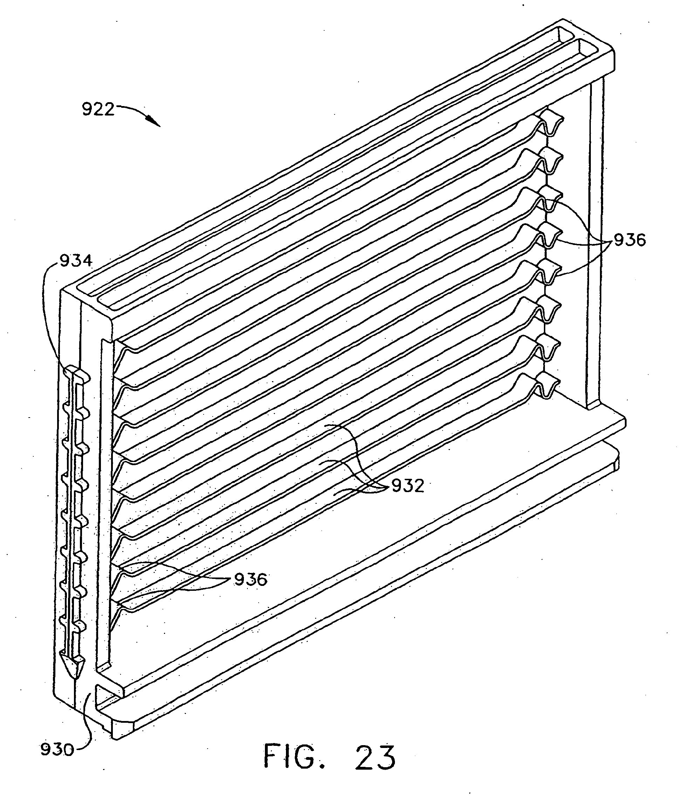

Referring to FIG. 23, one version of a compression assembly 922 is shown having a frame 930 with a plurality of compression members or compression slats 932 located therein. The frame 930 of the compression assembly 922 may be coupled with the localization fixture 16 via one or a plurality of connection members 934 for use during biopsy procedures. The connection members 934 may be configured such that the compression assembly 922 may be adjustable and/or detachable from the localization fixture 16 or other support.

The frame 930 may, for example, be rectangular in shape and have a plurality of adjustable and/or bendable compression slats 932 positioned therein. In the illustrated version, the compression slats 932 are generally rectangular in shape and include a flexible member 936 at each end thereof. The frame 930 may contain, for example, five compression slats 932 of equal size and shape distributed within the frame 930. The compression slats 932 and the frame 930 may be configured from any material, such as a polycarbonate stabilized for gamma irradiation, suitable for use during an MRI procedure or other medical procedure. It will be appreciated that the frame 930 and/or the compression slats 932 may be configured in any desirable shape or configuration facilitating access to a patient's breast during a biopsy procedure while still providing sufficient surface area to retain the breast securely therein.

Still referring to FIG. 23, the compression slats 932 may be coupled to the frame 930 permanently or detachably. The flexible members 936, in one version, are configured from an elastomeric material that allows the compression slats to be easily bent or adjusted to provide a wide range of access to the breast. The flexible members 936 may be integral with the compression slats 932 and may include, for example, folded or coiled memory-retention and/or polymeric material that may provide sufficient flexibility to the compression slats 932 such that they may be moved out of the way of a desired target area. The flexible members 936 may bias or otherwise return the compression slats 932 to their resting state when no longer displaced. In a further version, it is contemplated that the flexible members 936 may be permanently deformed when manipulated by the clinician. Although any suitable number is contemplated, each compression slat 932 may have a flexible member 936 at each end thereof adjacent the frame 930.

Still referring to FIG. 23, in use, the compression system 922 may be placed against the patient's breast with all of the compression slats 932 in their resting position, as depicted. Once the compression system 922 has been positioned and secured, the clinician may identify the region through which access to the breast would be most desirable. After identifying this area, the clinician may displace one or a plurality of compression slats 932 to provide the desired access. Once the compression slat 932 has been displaced, the clinician may access the breast with the biopsy device 14 via the newly created access point. In one version, the compression slat 932 may be configured to retain its newly acquired shape and/or position until it is otherwise moved.

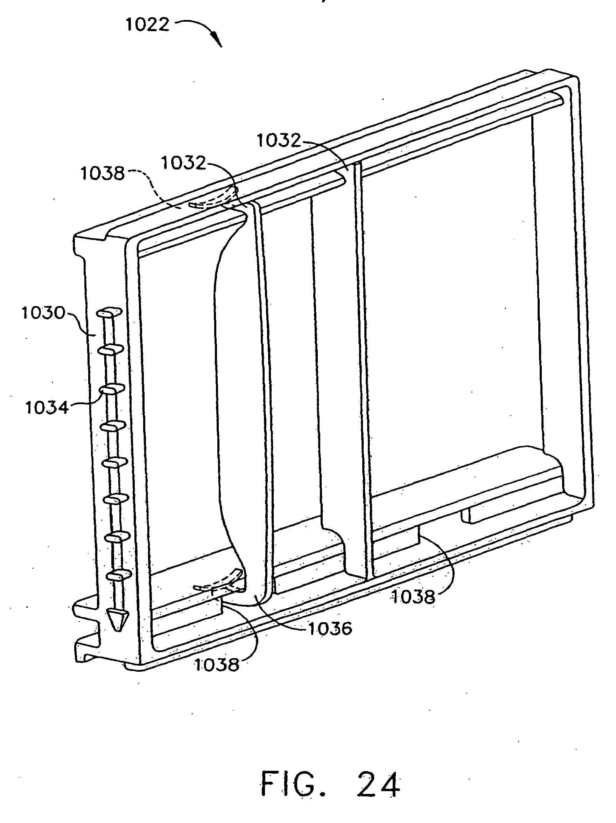

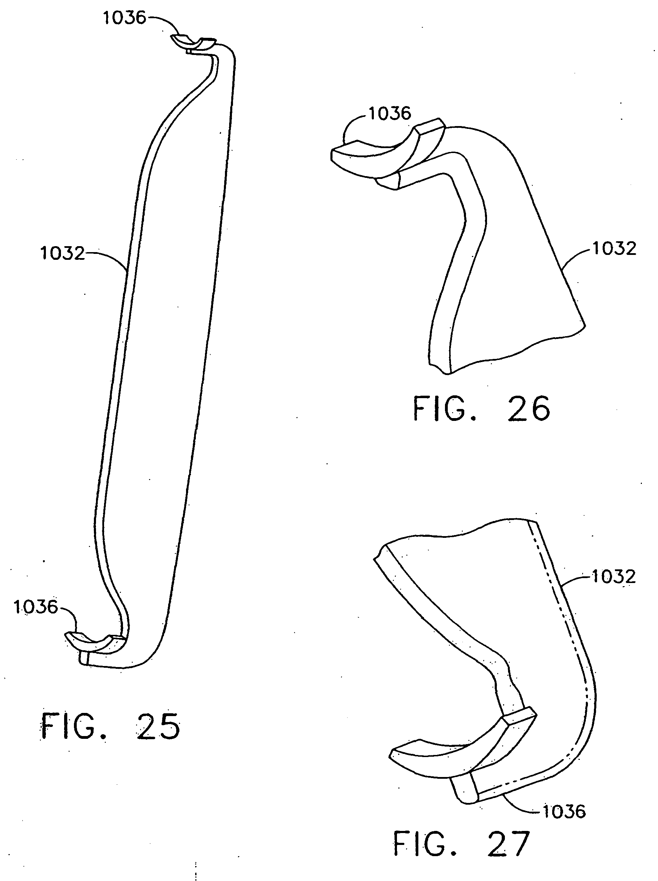

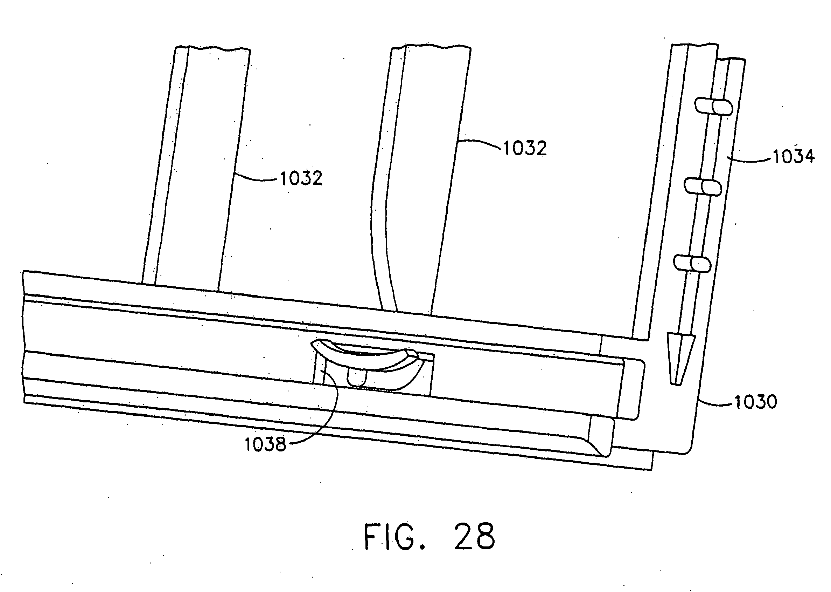

Referring to FIGS. 24-28, one version of a compression assembly 1022 is shown having a frame 1030 with a plurality of compression members or compression slats 1032 located therein. The frame 1030 of the compression assembly 1022 may be coupled with the localization fixture 16 via one or a plurality of connection members 1034 for use during biopsy procedures. The connection members 1034 may be configured such that the compression assembly 1022 may be adjustable and/or detachable from the localization fixture 16 or other support.

The frame 1030 may, for example, be rectangular in shape and have a plurality of adjustable, fixed, and/or removable compression slats 1032 positioned therein. In the illustrated version, the compression slats 1032 are generally longitudinal members and include a retainer 1036 at each end thereof. The frame 1030 may contain, for example, five compression slats 1032 of equal size and shape distributed within the frame 1030. In a further version, the frame 1030 may contain one or a plurality of fixed compression slats 1032 in addition to one or a plurality of adjustable and/or removable compression slats 1032. The compression slats 1032 and the frame 1030 may be configured from any material, such as a polycarbonate stabilized for gamma irradiation, suitable for use during an MRI procedure or other medical procedure. It will be appreciated that the frame 1030 and/or the compression slats 1032 may be configured in any desirable shape or configuration, including a vertical or horizontal configuration, facilitating access to a patient's breast during a biopsy procedure while still providing sufficient surface area to retain the breast securely therein.

Still referring to FIGS. 24-28, the compression slats 1032 may be coupled to the frame 1030 permanently or detachably. The retainers 1036, in one version, are configured as hooks retained within tracks 1038 to allow the compression slats to be removed, adjusted, and/or moved to provide a wide range of access to the breast. In one version, the retainer 1036 located at one end of the compression slat 1032 and engaged with the frame 1030 may have rotational, but little or no lateral movement capability, and the retainer 1036 at the opposite end may be free to move about a track 1038 or the like such that access points to a patient's breast may be exposed while providing structural support to the frame 1030. It will be appreciated that the retainers may take on any suitable shape or configuration that allows for the movement and/or detachment of the compression slats 1032. The retainers 1036 may be inserted into the tracks 1038 and may, in one version, have a range of motion therein. In further versions, the compression slats 1032 may be flexible, permanent, and/or removable. In the illustrated version, the retainers 1036 may be recessed away from the patient's breast to minimize the potential to pinch the skin.

Still referring to FIGS. 24-28, in use, the compression system 1022 may be placed against the patient's breast with all of the compression slats 1032 in place, as depicted. Once the compression system 1022 has been positioned and secured, the clinician may identify the region through which access to the breast would be most desirable. After identifying this area, the clinician may displace and/or remove one or a plurality of compression slats 1032 to provide the desired access. Once the compression slat 1032 has been displaced or removed, the clinician may access the breast with the biopsy device 14 via the newly created access point.

While preferred embodiments of the present invention have been shown and described herein, it will be obvious to those skilled in the art that such embodiments are provided by way of example only. Numerous variations, changes, and substitutions will now occur to those skilled in the art without departing from the scope of the appended claims. Additionally, each element described in relation to the invention may be alternatively described as a means for performing that element's function.

## Claims

1. A compression assembly (122) comprising:
(a) a frame (430); and
(b) a plurality of adjustable compression members (432) positioned within said frame (430), where said adjustable compression members (432) are engaged with said frame (430) such that at least one said adjustable compression member (432) is slidable therein, where adjusting at least one said compression member (432) opens an access point to a patient's breast, **characterised in that**:
said adjustable compression members (432) are substantially fan-shaped and are coupled at one pivot point and movable about a plurality of planes, where each compression member (432) is movable about a separate plane.

2. The compression assembly of claim 1, wherein said compression members (432) are engaged with said frame with at least one disengagable member.

3. The assembly of Claim 1 or 2, wherein said frame (430) is substantially rectangular in shape.

4. The assembly of any preceding claim, wherein said frame (430) and said adjustable compression members (432) are constructed from a polycarbonate stabilized for gamma irradiation.

5. The assembly of any preceding claim, wherein said adjustable compression members (432) are compression plates.

6. The assembly of any preceding claim, wherein said adjustable compression members (432) arc slidably or adjustably coupled, at one end, to the frame (430) with a hinge or the like.

7. The assembly of any preceding claim, wherein the compression members are stacked upon one another, and each compression member (432) is movable about a separate plane which runs parallel to a front face of the frame (430).

8. The assembly of any preceding claim, wherein the compression members (432) are freely adjustable about the plurality of planes and are held in place once positioned with a friction fit between adjacent surfaces of the movable members (432).

## Patentansprüche

1. Komprimierende Anordnung (122), die aufweist:
(a) einen Rahmen (430); und
(b) eine Vielzahl einstellbarer Kompressionselemente (432), die innerhalb des Rahmens (430) angeordnet sind, wobei die einstellbaren Kompressionselemente (432) derart im Eingriff mit dem Rahmen (430) sind, dass wenigstens eines der einstellbaren Kompressionselemente (432) darin verschieblich ist, wobei das Einstellen wenigstens eines der Kompressionselemente (432) einen Zugangspunkt zu der Brust eines Patienten öffnet, **dadurch gekennzeichnet, dass:**
die einstellbaren Kompressionselemente (432) im Wesentlichen fächerförmig sind und an einen Schwenkpunkt gekoppelt sind und um eine Vielzahl von Ebenen bewegbar sind, wobei jedes Kompressionselement (432) um eine getrennte Ebene bewegbar ist.

2. Komprimierende Anordnung nach Anspruch 1, bei der die Kompressionselemente (432) im Eingriff mit dem Rahmen sind, wobei wenigstens ein Element freisetzbar ist.

3. Anordnung nach Anspruch 1 oder 2, bei der der Rahmen (430) in der Form im Wesentlichen rechtwinklig ist.

4. Anordnung nach einem vorangehenden Anspruch, bei der der Rahmen (430) und die einstellbaren Kompressionselemente (432) aus einem Polykarbonat aufgebaut sind, das für die Gammabestrahlung stabilisiert worden ist.

5. Anordnung nach einem vorangehenden Anspruch, bei der die einstellbaren Kompressionselemente (432) Druckplatten sind.

6. Anordnung nach einem vorangehenden Anspruch, bei der die einstellbaren Kompressionselemente (432) mit einem Scharnier oder dergleichen verschieblich oder einstellbar mit einem Ende an den Rahmen (430) gekoppelt sind.

7. Anordnung nach einem vorangehenden Anspruch, bei der die Kompressionselemente aufeinander gestapelt sind und jedes Kompressionselement (432) um eine getrennte Ebene bewegbar ist, die parallel zu einer Vorderfläche des Rahmens (430) verläuft.

8. Anordnung nach einem vorangehenden Anspruch, bei der die Kompressionselemente (432) frei um die Vielzahl der Ebenen einstellbar sind und, sobald sie positioniert sind, mit einem Reibsitz zwischen benachbarten Flächen der bewegbaren Elemente (432) gehalten werden.

## Revendications

1. Ensemble de compression (122) comprenant :
(a) un cadre (430) ; et
(b) une pluralité d'éléments de compression ajustables (432) placés dans ledit cadre (430), lesdits éléments de compression ajustables (432) étant en prise avec ledit cadre (430) de telle sorte qu'au moins un élément de compression ajustable (432) puisse glisser dans celui-ci, lorsque l'ajustement d'au moins l'un desdits éléments de compression (432) ouvre un point d'accès dans le thorax d'un patient, **caractérisé en ce que** :
lesdits éléments de compression ajustables (432) sont substantiellement en forme de pales et sont couplés au niveau d'un pivot et sont mobiles sur une pluralité de plans, chaque élément de compression (432) étant mobile sur un plan séparé.

2. Ensemble de compression selon la revendication 1, dans lequel lesdits éléments de compression (432) sont en prise dans ledit cadre avec au moins un élément pouvant être ôté.

3. Ensemble selon la revendication 1 ou 2, dans lequel ledit cadre (430) est substantiellement de forme rectangulaire.

4. Ensemble selon l'une quelconque des revendications précédentes, dans lequel ledit cadre (430) et lesdits éléments de compression ajustables (432) sont constitués d'un polycarbonate stabilisé pour le rayonnement gamma.

5. Ensemble selon l'une quelconque des revendications précédentes, dans lequel lesdits éléments de compression ajustables (432) sont des plaques de compression.

6. Ensemble selon l'une quelconque des revendications précédentes, dans lequel lesdits éléments de compression ajustables (432) sont couplés de façon à glisser ou à être ajustés, à une extrémité, par rapport au cadre (430) avec une charnière ou autre.

7. Ensemble selon l'une quelconque des revendications précédentes, dans lequel les éléments de compression sont empilés les uns sur les autres et chaque élément de compression (432) est mobile sur un plan séparé qui est parallèle à une face frontale du cadre (430).

8. Ensemble selon l'une quelconque des revendications précédentes, dans lequel les éléments de compression (432) peuvent être ajustés librement autour de la pluralité de plans et sont maintenus en place une fois positionnés, avec un ajustement à pression entre les surfaces adjacentes des éléments mobiles (432).
